(19) Europäisches Patentamt
European Patent Office
Office européen des brevets

(11) Veröffentlichungsnummer : **0 100 479**
**B1**

(12) # EUROPÄISCHE PATENTSCHRIFT

(45) Veröffentlichungstag der Patentschrift : **13.03.85**

(21) Anmeldenummer : **83107010.7**

(22) Anmeldetag : **18.07.83**

(51) Int. Cl.⁴ : **C 07 C 87/38**, C 08 G 18/10, C 08 G 18/32, C 08 G 59/50, C 23 F 11/14// C07C45/49, C07C47/32

(54) **3-Aminomethyl-1-(3-aminopropyl-1-methyl)-4-methylcyclohexan, Verfahren zu seiner Herstellung und seine Verwendung.**

(30) Priorität : **31.07.82 DE 3228719**

(43) Veröffentlichungstag der Anmeldung : **15.02.84 Patentblatt 84/07**

(45) Bekanntmachung des Hinweises auf die Patenterteilung : **13.03.85 Patentblatt 85/11**

(84) Benannte Vertragsstaaten : **BE DE FR GB**

(56) Entgegenhaltungen : **FR-A- 2 346 315**

(73) Patentinhaber : **BAYER AG**
**Konzernverwaltung RP Patentabteilung**
**D-5090 Leverkusen 1 Bayerwerk (DE)**

(72) Erfinder : **Fiedler, Paul, Dr.**
**Rybniker Strasse 8**
**D-5000 Köln 80 (DE)**
Erfinder : **Braden, Rudolf, Dr.**
**Nothauserfeld 1**
**D-5068 Odenthal (DE)**

**EP 0 100 479 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die neue chemische Verbindung 3-Aminomethyl-1-(3-aminopropyl-1-methyl)-4-methyl-cyclohexan, ein Verfahren zu ihrer Herstellung, bei dem ausgehend von Limonen und/oder Monohydroformylierungsprodukten des Limonens zunächst ein Dihydroformylierungsprodukt des Limonens und daraus die erfindungsgemäße Verbindung erhalten wird, sowie die Verwendung der neuen Verbindung als Korrosionsschutzmittel, als Vernetzer für Epoxidharze und als Kettenverlängerungsmittel für NCO-Prepolymere.

Die neue chemische Verbindung 3-Aminomethyl-1-(3-aminopropyl-1-methyl)-4-methyl-cyclohexan kann mit der Formel

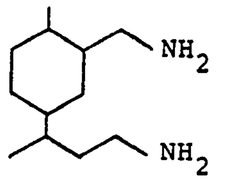

gekennzeichnet werden und wird im folgenden auch als erfindungsgemäßes Diamin bezeichnet.

Von dem erfindungsgemäßen Diamin existieren acht verschiedene diastereomere Formen, die normalerweise als Gemisch aller oder einzelner Diastereomere vorliegen. Aus solchen Gemischen können die einzelnen Diastereomeren durch übliche Methoden der Diastereomerentrennung angereichert oder isoliert werden, beispielsweise durch Kapillar-Gaschromatographie (Kapillar-GC) oder Hochdruck-Flüssigkeits-Chromatographie (HPLC).

Das erfindungsgemäße Verfahren zur Herstellung von 3-Aminomethyl-1-(3-aminopropyl-1-methyl)-4-methyl-cyclohexan ist dadurch gekennzeichnet, daß man Limonen und/oder Monohydroformylierungsprodukte des Limonens in Gegenwart eines Rhodium enthaltenden Katalysators, der außer Rhodium wenigstens einen Liganden aus der Gruppe Kohlenmonoxid, der Alkene mit 2 bis 12 C-Atomen und der Stickstoff, Schwefel, Sauerstoff oder Phosphor enthaltenden organischen Verbindungen enthält, in einer Menge, die weniger als 1 g Rhodium pro kg Ausgangsmaterial entspricht, mit Kohlenmonoxid und Wasserstoff bei Temperaturen von 80 bis 180 °C und Drucken von 20 bis 1 000 bar umsetzt und anschließend das erhaltene Dihydroformylierungsprodukt des Limonens in Gegenwart von Ammoniak und einem Hydrierkatalysator bei 50 bis 150 °C mit Wasserstoff behandelt.

In das erfindungsgemäße Verfahren kann Limonen (4-Isopropenylmethylcyclohexen) beliebiger Herkunft eingesetzt werden. Limonen kann in Form von Enantiomerengemischen z. B. d,l-Limonen, als Enantiomerengemisch, in dem ein Enantiomeres angereichert ist oder in Form eines reinen Enantiomeren, z. B. als (R), (+)-Limonen oder (S), (−)-Limonen eingesetzt werden. Limonen ist beispielsweise durch Extraktion aus Naturstoffen, insbesondere aus Zitrusfrüchten und bestimmten Koniferenarten, zugänglich.

In das erfindungsgemäße Verfahren können auch Monohydroformylierungsprodukte des Limonens eingesetzt werden, beispielsweise 4-Methyl-cyclohex-3-en-β-methyl-propanal. Die Monohydroformylierung des Limonens ist bekannt, s. z. B. K. Kogami, Japan Yakagaku 22 (6), S. 316 (1973). Die Monohydroformylierungsprodukte des Limonens können als Reaktionsgemisch, wie es bei der Monohydroformylierung von Limonen, vorzugsweise nach Abtrennung des dabei verwendeten Katalysators, anfällt, eingesetzt werden. Geeignet sind auch Monohydroformylierungsprodukte des Limonens, die nach destillativer Aufarbeitung des Reaktionsgemisches aus der Monohydroformylierung von Limonen anfallen.

Weiterhin können beliebige Gemische aus Limonen und Monohydroformylierungsprodukten des Limonens in das erfindungsgemäße Verfahren eingesetzt werden.

Die erfindungsgemäße Umsetzung von Limonen und/oder Monohydroformylierungsprodukten des Limonens mit Kohlenmonoxid und Wasserstoff erfolgt in Gegenwart eines Rhodium enthaltenden Katalysators, der außer Rhodium wenigstens einen Liganden aus der Gruppe Kohlenmonoxid, der Alkene mit 2 bis 12 C-Atomen und der Stickstoff, Schwefel, Sauerstoff oder Phosphor enthaltenden organischen Verbindungen enthält. Vorzugsweise werden solche Rhodium enthaltenden Katalysatoren verwendet, die im Reaktionsgemisch löslich sind. Die Rhodium enthaltenden Katalysatoren können auch mehrere gleichartige oder verschiedenartige Liganden der vorgenannten Art enthalten, sowie zusätzlich anionische Reste, beispielsweise Halogenatome und/oder Acetatreste.

Geeignete Liganden aus der Gruppe der Alkene mit 2 bis 12 C-Atomen können offenkettig, cyclisch oder bicyclisch sein und eine oder mehrere olefinische Doppelbindungen enthalten. Vorzugsweise enthalten solche Liganden zwei olefinische Doppelbindungen. Als Beispiele seien Bicyclo[2.2.1]hepta-1,4-dien und 1,5-Cyclooctadien genannt.

Geeignete Liganden aus der Gruppe der Stickstoff enthaltenden organischen Verbindungen sind beispielsweise Amine. Bevorzugt sind tertiäre Amine, insbesondere aromatische und heterocyclische

tertiäre Amine. Als Beispiele seien Pyridin, Picoline, Ethylpyridine, N-Methylpyrrolidin, N-Methylpyrrol, N,N'-Dimethylpiperazin, Dimethylcyclohexylamin, Triethylamin, N,N-Dimethylanilin, N-Methylmorpholin, N-Methylindol, Chinolin, Isochinolin und N-Methylpyrrolidon genannt.

Geeignete Liganden aus der Gruppe der Schwefel enthaltenden organischen Verbindungen sind beispielsweise organische Disulfide und Sulfoxide. Als Beispiele seien Dibenzylsulfid, Di-n-butylsulfid, Dimethylsulfoxid, Diethylsulfoxid, Di-(4-chlor-benzyl)-sulfid, Di-(4-cyanobenzyl)-sulfid, Bis-(4-dimethylaminobenzyl)-sulfid, Di-(4-diethylaminobenzyl)-sulfid, Di-($\alpha$-napthylmethyl)-sulfid, Di-(2,6-dichlorbenzyl)-sulfid, Di-(3,4-dichlorbenzyl)-sulfid, Di-(2-chlorbenzyl)-sulfid, Di-(5,6,7,8-tetrahydronaphthyl-2-methyl)-sulfid, Benzyl-methyl-sulfid, Benzyl-dodecyl-sulfid, 4-Dimethylaminobenzyl-methyl-sulfid, Benzyl-butylsulfid, Bis-(4-carboxybenzyl)-sulfid, Di-(4-methylbenzyl)-sulfid, Di-(3-methylbenzyl)-sulfid und Di-(2-methylbenzyl)-sulfid genannt.

Geeignete Liganden aus der Gruppe der Sauerstoff enthaltenden organischen Verbindungen sind beispielsweise unsubstituierte und substituierte Acetylacetonate. Beispiele für substituierte Acetylacetonate sind solche, die Alkyl-, Phenyl-, substituierte Phenyl- und/oder Fluoralkylgruppen enthalten. Als Beispiele für Liganden aus der Gruppe Sauerstoff enthaltender organischer Verbindungen seien, 1,1,1-Trifluor-2,4-pentadion, Benzoylaceton und 2-Acetylcyclopentanon genannt.

Geeignete Liganden aus der Gruppe der Phosphor enthaltenden organischen Verbindungen sind beispielsweise Triorganophosphorliganden. Besonders geeignet sind tertiäre organische Phosphine und Phosphite, die gleiche oder verschiedene Alkylreste mit 1 bis 20 C-Atomen, Cycloalkylreste mit 5 bis 12 C-Atomen, Aralkylreste mit 7 bis 10 C-Atomen und mindestens einen Arylrest mit 3 bis 10 C-Atomen aufweisen. Diese Alkyl-, Cycloalkyl-, Aralkyl- und Arylreste können Substituenten aufweisen, z. B. 1 bis 3 Hydroxylgruppen, Alkoxy- und/oder Carboalkoxygruppen mit 1 bis 4 C-Atomen, gegebenenfalls substituierte Aminogruppen und/oder Halogenatome. Als Beispiele für Liganden aus der Gruppe Phosphor enthaltender organischer Verbindungen seien Triphenylphosphin, Diethylphenylphosphin, Tritolylphosphin, Trinaphthylphosphin, Diphenylmethylphosphin, Diphenylbutylphosphin, Tris-(p-chlorphenyl)-phosphin, Tris-(p-carbmethoxyphenyl)-phosphin, Tris-(p-cyanophenyl)-phosphin, Diphenylphosphonig-säurephenylester, Benzolphosphonigsäure-di-phenylester und Triphenylphosphit, $P[CH_2CH_2CH_2N(CH_3)_2]_3$, $P[CH_2CH_2CH_2N(C_2H_5)_2]_3$, $P(CH_2CH_2CH_2\text{-NH-iso-}C_4H_9)_3$, $P[CH_2CH_2CH_2N(\text{iso-}C_4H_9)_2]_3$, $(n\text{-}C_4H_9)_2PCH_2CH_2N(C_2H_5)_2$, $P[CH_2N(C_2H_5)_2]_3$, $P[C_6H_4N(CH_3)_2]_3$, $P[CH_2CH_2C_6H_4N(C_2H_5)_2]_3$,

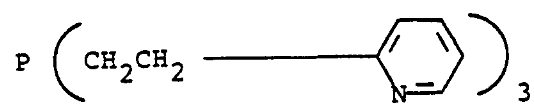

$P[CH_2CH_2CH_2N(\text{tert.}C_4H_9)_2]_3$ und $P[CH_2CH_2CH_2N(\text{iso-}C_3H_7)_2]_3$ genannt.

Von den tertiären organischen Phosphinen und Phosphiten sind die Triarylphosphine und die Triarylphosphite bevorzugt.

Geeignete Liganden aus der Gruppe der Phosphor enthaltenden organischen Verbindungen sind auch Komplexligande, die aus teilweise durch Ferrocen substituierten Triorganophosphinen bestehen, wie sie beispielsweise in der DE-OS 2 617 306 beschrieben sind.

Bevorzugte Rhodium enthaltende Katalysatoren für das erfindungsgemäße Verfahren sind solche, die einer der Formeln

$$XRh(CO)L_2,\ XRh(CO)L_3,\ RhXL_3,\ [Rh(CO)_2L_2]_2 \text{ und } [Rh(OCOCH_3)L]_2$$

entsprechen, in denen X ein Chlor-, Brom- oder Jodatom und L einen der zuvor beschriebenen Liganden bedeutet.

Besonders bevorzugte Rhodium enthaltende Katalysatoren für das erfindungsgemäße Verfahren sind dimeres Rhodiumcarbonylchlorid, $Rh_2(C_8H_{12})_2Cl_2$, $Rh(CO)_2$ acac, $Rh[[(C_5H_5)_2Fe]PPh_2]_3Cl_3$, $Rh[(C_6H_5CH_2)_2S]_3Cl_3$, $Rh(PPh_3)_3Cl$, $Rh(PPh_3)_3Br$, Rh(PPh-3)-3Cl-3, Rhodium-2-ethylhexanoat, $Rh[(4\text{-}CH_3O\text{-}C_6H_4CH_2)_2S]_3Cl_3$, $Rh[(4\text{-}Cl\text{-}C_6H_4CH_2)_2S]Cl_3$, $Rh[(C_6H_5CH_2)_2SO]_3Cl_3$ und $Rh[(CH_3)_2SO]_3Cl_3$. acac steht hierbei für einen Acetylacetonatrest und Ph für eine Phenylgruppe.

Die zuvor beschriebenen Rhodium enthaltenden Katalysatoren brauchen nicht als solche in das erfindungsgemäße Verfahren eingesetzt zu werden. Es kann vorteilhaft sein, ein Rhodiumsalz und einen oder mehrere Liganden getrennt dem erfindungsgemäßen Verfahren zuzuführen und somit auf eine separate Herstellung von Rhodium-Komplexverbindungen zu verzichten. Weiterhin ist es im allgemeinen möglich, einen Teil des Rhodiums durch Cobalt zu ersetzen. Im allgemeinen kann man bis zu 95 Gew.-% des Rhodiums, bevorzugt bis zu 90 Gew.-% des Rhodiums, durch Cobalt ersetzen.

Die Rhodium enthaltenden Katalysatoren werden erfindungsgemäß in einer Menge eingesetzt, die

weniger als 1 g Rhodium (berechnet als Metall) pro kg eingesetztem Limonen und/oder Monohydroformylierungsprodukten des Limonens (= Ausgangsprodukt) entspricht. Bevorzugt werden 1 bis 1 000 mg, besonders bevorzugt 10 bis 600 mg Rhodium (berechnet als Metall) pro kg Ausgangsprodukt eingesetzt.

Die erfindungsgemäße Hydroformylierung wird i. a. in flüssiger Phase durchgeführt. Dabei kann der Rhodium enthaltende Katalysator auch auf einem festem Träger fixiert sein, wie es beispielsweise von P. I. Davidson et al in Catalysis, Vol. 1 (1976), S. 391-395 beschrieben ist.

Im einfachsten Fall stellt die flüssige Phase ein Gemisch der vorhandenen Stoffe (Ausgangsprodukt(e), Reaktionsprodukt(e), Rhodium enthaltender Katalysator) dar. Es kann aber auch in Gegenwart eines oder mehrerer Lösungsmittel gearbeitet werden, die unter Reaktionsbedingungen inert oder weitgehend inert sind und den Rhodium enthaltenden Katalysator zu lösen vermögen. Beispiele für geeignete Lösungsmittel sind alkylsubstituierte Benzole, höhersiedende Ester wie Dialkyldicarboxylate, Ester von Polyolen wie Ester von Trimethylolpropan oder Pentaerythrit, höhersiedende Ketone, höhersiedende Alkohole wie Butanole, höhersiedende Ether, höhersiedende Kohlenwasserstoffe wie gesättigte aliphatische und cycloaliphatische Kohlenwasserstoffe.

Wenn Lösungsmittel eingesetzt werden sind solche bevorzugt, die auch für die nachfolgende Behandlung mit Wasserstoff in Gegenwart von Ammoniak geeignet sind. Als Beispiele hierfür seien Benzol, Toluol, Xylol, Isopropanol, Methylcyclohexan, Dekalin, Dioxan, Tetrahydrofuran, Ethylenglykolmonoethylether und Diethylenglykoldimethylether genannt.

Bei der erfindungsgemäßen Umsetzung von Limonen und/oder Monohydroformylierungsprodukten des Limonens mit Kohlenmonoxid und Wasserstoff werden Kohlenmonoxid und Wasserstoff im allgemeinen mindestens in solchen Mengen eingesetzt, die theoretisch für die vollständige Bildung eines Dihydroformylierungsproduktes des Limonens erforderlich sind. Vorzugsweise setzt man Kohlenmonoxid und Wasserstoff im Überschuß ein, beispielsweise in einem Überschuß von jeweils bis zu 1 000 Mol-%. Kohlenmonoxid und Wasserstoff können in verschiedenen Verhältnissen zueinander eingesetzt werden. Das Volumenverhältnis von Kohlenmonoxid zu Wasserstoff kann beispielsweise von 4 : 1 bis 1 : 4 betragen. Bevorzugt beträgt dieses Verhältnis 2 : 1 bis 1 : 1. Besonders bevorzugt werden etwa gleiche Volumenmengen Kohlenmonoxid und Wasserstoff eingesetzt.

Die erfindungsgemäße Umsetzung von Limonen und/oder Monohydroformylierungsprodukten des Limonens mit Kohlenmonoxid und Wasserstoff wird bei Temperaturen von 80 bis 180 °C und Drucken von 20 bis 1 000 bar durchgeführt. Vorzugsweise liegt die Temperatur im Bereich 105 bis 180 °C und der Druck im Bereich 100 bis 400 bar. Temperaturen im Bereich 120 bis 170 °C sind besonders bevorzugt.

Es kann vorteilhaft sein, die Hydroformylierung stufenweise zunächst bei einer tieferen Temperatur, z. B. bei 80 bis 140 °C, und anschließend bei einer höheren Temperatur, z. B. 130 bis 180 °C, durchzuführen. Ebenso kann es vorteilhaft sein, den Katalysator, das Lösungsmittel und/oder das Ausgangsmaterial zum Teil erst während der Reaktion zuzuführen.

Das nach der erfindungsgemäßen Umsetzung von Limonen und/oder Monohydroformylierungsprodukten des Limonens mit Kohlenmonoxid und Wasserstoff vorliegende Reaktionsgemisch kann direkt in die Behandlung mit Wasserstoff in Gegenwart von Ammoniak (reduktive Aminierung) eingesetzt werden. Insbesondere ist es nicht erforderlich, den Rhodium enthaltenden Katalysator abzutrennen, was unter wirtschaftlichen Gesichtspunkten vorteilhaft sein kann, weil die Abtrennung der sehr geringen Katalysatormengen aufwendig sein kann. Man kann aus dem Reaktionsgemisch aber auch den Katalysator abtrennen, z. B. durch Destillation oder Extraktion, und ihn gegebenenfalls wieder verwenden. Weiterhin kann es vorteilhaft sein, aus dem Reaktionsprodukt das Dihydroformylierungsprodukt des Limonens abzutrennen, z. B. durch Destillation, und nur dieses in die folgende reduktive Aminierung einsetzen. Dabei können gegebenenfalls noch vorhandene Monohydroformylierungsprodukte ebenfalls abgetrennt und gegebenenfalls einer erneuten Umsetzung mit Kohlenmonoxid und Wasserstoff zugeführt werden.

Die erfindungsgemäße reduktive Aminierung wird im allgemeinen in Gegenwart von mindestens 2 Mol Ammoniak pro Mol Dihydroformylierungsprodukt des Limonens durchgeführt. Vorzugsweise wird ein Überschuß an Ammoniak eingesetzt, beispielsweise 3 bis 20 Mol Ammoniak pro Mol Dihydroformylierungsprodukt des Limonens.

Die erfindungsgemäße reduktive Aminierung wird in Gegenwart eines Hydrierkatalysators durchgeführt. Geeignete Katalysatoren sind beispielsweise solche, die als Aktivkomponente eines oder mehrere der Elemente mit den Ordnungszahlen 23 bis 29 in metallischer und/oder oxidischer Form enthalten.

Geeignete Katalysatoren sind beispielsweise Nickel- oder Kobalt-Katalysatoren, wie Nickel-auf-Träger, wobei als Träger anorganische Materialien wie Kieselgur, Kieselsäuren, Aluminiumoxide, Silikate, Aluminiumsilikate, Montmorillonit, Zeolithe, Spinelle, Dolomit, Kaolin, Magnesiumsilikate, Zirkonoxid, Eisenoxid, Zinkoxid, Calciumcarbonat, Siliziumcarbid, Aluminiumphosphat, Borphosphat, Asbest oder Aktivkohle und als organische Katalysatorträger natürlich vorkommende oder synthetische Verbindungen mit hohem Molgewicht wie Seide, Polyamide, Polystyrole, Zellstoff oder Polyurethane verwendbar sind. Die Träger können z. B. in Form von Kugeln, Strägen, Fäden, Zylindern, Polygonen oder in Pulverform vorliegen. Weitere geeignete Katalysatoren sind Raney-Typ-Katalysatoren, wie Raney-Nickel, W-1-, W-5-, W-6-, W-7-Raney-Nickel wie von H. Adkins, J. Am. Chem. Soc. *69,* 3039 (1974) beschrieben, Raney-Kobalt-Katalysatoren, Raney-Kupfer, Raney-Nickel-Eisen, Raney-Kobalt-Nickel, Raney-Kobalt-Eisen, sowie durch Reduktion von Nickel- oder Kobaltsalzen hergestellte Metallkatalysatoren,

wie Urushibara-Nickel oder mit Metallalkylverbindungen, Alkalihydriden, Hydrazin, Boranaten oder Borwasserstoff reduzierte Nickel- oder Kobaltsalze, durch Reduktion der Metalloxide oder Metalloxidgemische hergestellte Katalysatoren und die Metalloxide oder -oxidgemische.

Die Reduktion der Metalloxide oder Metallsalze kann auch mit Wasserstoff, gegebenenfalls bei erhöhter Temperatur und erhöhtem Druck, unter den Bedingungen des Verfahrens oder während des Verfahrens geschehen.

Die Katalysatoren können als Beschleuniger eines oder mehrerer der folgenden Elemente in Mengen bis zu 10 Gew.-% enthalten : Li, Na, Ca, Ba, K, Ag, Be, La, Ce, Ti, V, Nb, Ta, Mo, W, und bis zu 1 % der Elemente Ru, Rh, Pd, Au, Ir, Pt.

Besonders bevorzugte Hydrierkatalysatoren sind Raney-Katalysatoren, wie Raney-Nickel, Raney-Kobalt und Raney-Nickel-Eisen.

Im allgemeinen werden 0,01 bis 10 Gew.-% Hydrierkatalysator eingesetzt. Bevorzugt ist der Einsatz von 0,1 bis 5 Gew.-% Hydrierkatalysator.

Die erfindungsgemäße reduktive Aminierung wird bei 50 bis 150 °C durchgeführt. Temperaturen von 90 bis 135 °C sind bevorzugt. Der Wasserstoffdruck während der Aminierung soll im allgemeinen über 10 bar betragen. Wasserstoffdrucke von 50 bis 200 bar sind bevorzugt.

Die erfindungsgemäße reduktive Aminierung kann in Gegenwart oder Abwesenheit von Lösungsmitteln durchgeführt werden. Geeignete Lösungsmittel sind weiter oben beschrieben. Man kann das Reaktionsgemisch auch mit dem erfindungsgemäßen Diamin verdünnen. Vorzugsweise arbeitet man bei einem Verhältnis von Dihydroformylierungsprodukt zu Lösungsmittel von 1 zu 3 bis 3 zu 1 Gewichtsteilen.

Es ist möglich, die erfindungsgemäße reduktive Aminierung kontinuierlich und diskontinuierlich durchzuführen.

Es kann vorteilhaft sein, aus dem Dihydroformylierungsprodukt des Limonens und Ammoniak, gegebenenfalls in Gegenwart eines Lösungsmittels, zunächst die Schiff'sche Base herzustellen, das dabei freiwerdende Wasser abzutrennen, beispielsweise durch Phasentrennung, und erst anschließend die Behandlung mit Wasserstoff durchzuführen. Weiterhin kann es vorteilhaft sein, in einem Hydrierautoklaven den Hydrierkatalysator, Ammoniak und ein Lösungsmittel oder bereits hergestelltes erfindungsgemäßes Diamin unter Wasserstoffdruck vorzulegen und das Dihydroformylierungsprodukt des Limonens, gegebenenfalls zusammen mit einem Lösungsmittel, dazuzupumpen. Schließlich kann es auch vorteilhaft sein, die erfindungsgemäße reduktive Aminierung unter Zusatz einer katalytischen Menge einer Säure durchzuführen. Solche Säuren können anorganische Säuren sein, wie Phosphorsäure, aber auch organische Säuren, wie Essigsäure, Propionsäure oder Bernsteinsäure. Bevorzugt sind Zusätze an Säuren in Mengen von 0,1 bis 3 Gew.-% (bezogen auf eingesetztes Dihydroformylierungsprodukt).

Die erfindungsgemäße reduktive Aminierung verläuft normalerweise sehr selektiv. Beispielsweise werden dabei 90 bis 99 % des eingesetzten Limonendialdehyds in das erfindungsgemäße Diamin überführt. Wenn man reinen oder weitgehend reinen Limonendialdehyd in die reduktive Aminierung eingesetzt hat, so kann man deshalb ausreichend reines erfindungsgemäßes Diamin erhalten, wenn man nach der reduktiven Aminierung lediglich den hydrierkatalysator, z. B. durch Filtration oder Zentrifugation, und gegebenenfalls das Lösungsmittel abtrennt. In anderen Fällen, insbesondere, wenn in die reduktive Aminierung ein rohes Dihydroformylierungsprodukt des Limonens eingesetzt wurde, ist es im allgemeinen erforderlich, das Reaktionsgemisch nach der Abtrennung des Hydrierkatalysators weiter aufzuarbeiten, beispielsweise durch Destillation oder Rektifikation, um ein ausreichend reines erfindungsgemäßes Diamin zu erhalten.

Wie eingangs beschrieben fällt das erfindungsgemäße Diamin im allgemeinen als Gemisch einzelner oder aller enantiomerer Formen an. Die Zusammensetzung dieses Gemisches hängt u. a. vom Ausgangsmaterial ab, insbesondere davon, ob R (+)-Limonen, S (+)-Limonen, d,l-Limonen oder ein bestimmtes Gemisches dieser enantiomeren Limonene eingesetzt wurde.

Die vorliegende Erfindung betrifft weiterhin die Verwendung von 3-Aminomethyl-1-(3-aminopropyl-1-methyl)-4-methyl-cyclohexan als Korrosionsschutzmittel, Vernetzer für Epoxidharze und Kettenverlängerungsmittel für NCO-Prepolymere, die z. B. zur Herstellung von Polyurethan-Polyharnstoffen eingesetzt werden. Das erfindungsgemäße Diamin weist eine ausgezeichnete Verträglichkeit mit Heizölen, Schmierstoffen und Treibmitteln auf Kohlenwasserstoffbasis auf und kann deshalb solchen Stoffen mit Vorteil als Korrosionsschutzmittel zugefügt werden, beispielsweise in Mengen von 0,01 bis 1 Gew.-%. Bei Verwendung des erfindungsgemäßen Diamins als Vernetzer für Epoxidharze oder Kettenverlängerungsmittel für NCO-Prepolymere wird es beispielsweise in solchen Mengen angewendet, wie andere Diamine für diesen Zweck eingesetzt werden. Für die angegebenen Verwendungen des erfindungsgemäßen Diamins ist es ohne Bedeutung, in welchem Diastereomerengemisch es vorliegt.

Mit der vorliegenden Erfindung wird erstmals ein technisch interessantes Diamin zur Verfügung gestellt, das aus dem nachwachsenden Rohstoff Limonen zugänglich ist.

Es ist ausgesprochen überraschend, das es erfindungsgemäß gelingt, das 3-Aminomethyl-1-(3-aminopropyl-1-methyl)-4-methyl-cyclohexan in guten Ausbeuten aus Limonen zu erhalten. Bisher ist insbesondere kein technisch brauchbares Verfahren zur Herstellung von Dihydroformylierungsprodukten des Limonens bekannt geworden.

So beschreibt K. Kogami, Japan Yakagaku 22 (6), S. 316 (1973) ein Verfahren zur Hydroformylierung von Limonen, bei dem beim Einsatz von Kobaltcarbonyl als Katalysator in 45 bis 52 %iger Ausbeute 4-

Methylcyclohex-3-en-β-methylpropanal (das ist ein Monohydroformylierungsprodukt des Limonens) und in max. 16 %iger Ausbeute das Dihydroformylierungsprodukt des Limonens anfällt. Wenn beim gleichen Verfahren als Katalysator Rhodiumtrichlorid × $3H_2O$ in einer Menge von 1,7 bis 3,4 g Rhodium pro kg Limonen eingesetzt wird, erhält man das Dihydroformylierungsprodukt des Limonens nur in einer Ausbeute von 2 bis 7 %.

In Ind. Eng. Chem. Proc. Res. Dev. *4*, 283 (1965) sind Hydroformylierungen von Limonen in verschiedenen Lösungsmitteln beschrieben. Es werden immer komplexe Gemische der Monohydroformylierungsprodukte erhalten, die nur wenig Dihydroformylierungsprodukt enthalten.

Die EP-OS 54 986 zeigt zwar eine Verbesserung der Herstellung von 4-Methylcyclohex-3-en-β-methylpropanal, gibt jedoch keine Lehre, wie man die zweite im Limonen vorhandene Doppelbindung, das ist die durch eine Methylgruppe substituierte Doppelbindung im Ring, hydroformylieren kann.

Auch E. Falbe, New Syntheses with Carbon Monoxide, Springer-Verlag Heidelberg, S. 117 (1980) kann nicht entnommen werden, wie man Dihydroformylierungsprodukte des Limonens erhalten kann.

Bei diesem Stand der Technik bestand für den Fachmann ein erhebliches Vorurteil gegen die Herstellung des erfindungsgemäßen Diamins aus Limonen über die Zwischenstufe der Dihydroformylierungsprodukte des Limonens zu versuchen. Keinesfalls konnte erwartet werden, daß Dihydroformylierungsprodukte des Limonens mit gegenüber der Literaturstelle K. Kogami geringeren Mengen eines modifizierten Rhodiumkatalysators in Selektivitäten von über 75 % und daraus erfindungsgemäße Diamin in Ausbeuten von über 90 % zugänglich sind.

## Beispiele

### Beispiel 1

a) Hydroformylierung

100 g Limonenmonoaldehyd (4-Methylcyclohex-3-en-β-methylpropanal), 300 ml Cyclohexan und 0,1 g dimeres Rhodiumcarbonylchlorid wurden in einen Edelstahlautoklaven von 700 ml Inhalt gegeben und unter Rühren und unter Wassergas ($CO/H_2$) auf 145 °C erhitzt. Durch regelmäßiges Nachdrücken von Wassergas wurde der Druck zwischen 200 und 220 bar gehalten. Nach 2 Stunden blieb der Druck konstant. Es wurde abgekühlt, entspannt und das Reaktionsgemisch durch Dünnschichtdestillation aufgetrennt. Bei 150 °C und 0,05 mbar wurden 75 g Limonendialdehyd (4-Methyl-3-formylcyclohexan-β-methylpropanal) erhalten, was einer Selektivität von 75 % entspricht. IR ($cm^{-1}$) 2 920, 2 720, 1 725, 1 450, 1 380.

b) Reduktive Aminierung

In einem Edelstahlautoklaven von 1,3 l Inhalt wurden 135 g Limonendialdehyd (4-Methyl-3-formylcyclohexan-β-methylpropanal), 300 ml Tetrahydrofuran und 10 g Raney-Nickel vorgelegt und gerührt. Nach Aufdrücken von 300 ml flüssigem Ammoniak wurde auf 100 °C erhitzt und diese Temperatur während 1 Stunde gehalten. Anschließend wurde bei 120 °C und 140 bar Gesamtdruck mit Wasserstoff hydriert. Durch regelmäßiges Nachdrücken von Wasserstoff wurde der Druck auf 140 bar gehalten. Nach 3 Stunden blieb der Druck konstant. Es wurde abgekühlt, entspannt, der Katalysator abgetrennt und das Reaktionsgemisch destillativ in seine Komponenten aufgetrennt. Bei 147 bis 156 °C und 0,05 mbar Druck wurden 117 g 3-Aminomethyl-1-(3-aminopropyl-1-methyl)-4-methyl-cyclohexan erhalten, was einer Selektivität von 87 % entspricht. Durch Titration des Diamins mit 1n HCl wurde das Äquivalentgewicht bestimmt, das 100,2 g pro Mol betrug (berechnet : 99 g pro Mol). IR ($cm^{-1}$) 3 300, 2 900, 1 600, 1 450, 1 380, 830.

### Beispiel 2

300 g Limonen, 500 ml Cyclohexan und 0,3 g $Rh_2(C_8H_{12})_2Cl_2$ wurden in einem 1,3 l-Edelstahlautoklaven unter Rühren und unter Wassergasdruck auf 150 °C erhitzt. Durch regelmäßiges Nachdrücken von Wassergas wurde der Druck zwischen 200 und 220 bar gehalten. Nach beendeter Reaktion wurde abgekühlt, entspannt und das Reaktionsgemisch destillativ aufgetrennt. Die geringen Anteile an erhaltenem Monoaldehyd (Kp 90 °C/0,1 mbar) wurden wie in Beispiel 1a beschrieben weiter hydroformyliert. Der erhaltene Dialdehyd (Kp 150 °C/0,05 mbar) wurde wie in Beispiel 1b beschrieben weiterverarbeitet.

### Beispiel 3

250 g Limonenmonoaldehyd, 750 ml Toluol und 0,26 g Rh $[[(C_5H_5)_2Fe]PPh_2]_3\ Cl_3$ wurden in einem Edelstahlautoklaven von 1,6 l Inhalt unter Rühren und Wassergasdruck ($CO/H_2$ 1 : 1) auf 145 °C erhitzt. Durch regelmäßiges Nachdrücken von Wassergas wurde der Druck zwischen 250 und 280 bar gehalten. Nach etwa 2 h blieb der Druck konstant, es wurde abgekühlt und entspannt. Die Gaschromatographie

Analyse ergab einen Gehalt an Dialdehyd von 21 % was einer Ausbeute von 70 % und einer Selektivität von 81 % entspricht.

## Ansprüche

1. 3-Aminomethyl-1-(3-aminopropyl-1-methyl)-4-methylcyclohexan.

2. Verfahren zur Herstellung von 3-Aminomethyl-1-(3-aminopropyl-1-methyl)-4-methyl-cyclohexan, dadurch gekennzeichnet, daß man Limonen und/oder Monohydroformylierungsprodukte des Limonens in Gegenwart eines Rhodium enthaltenden Katalysators, der außer Rhodium wenigstens einen Liganden aus der Gruppe Kohlenmonoxid, der Alkene mit 2 bis 12 C-Atomen und der Stickstoff, Schwefel, Sauerstoff oder Phosphor enthaltenden organischen Verbindungen enthält, in einer Menge, die weniger als 1 g Rhodium pro kg Ausgangsmaterial entspricht, mit Kohlenmonoxid und Wasserstoff bei Temperaturen von 80 bis 180 °C und Drucken von 20 bis 1 000 bar umsetzt und anschließend das erhaltene Dihydroformylierungsprodukt des Limonens in Gegenwart von Ammoniak und einem Hydrierkatalysator bei 50 bis 150 °C mit Wasserstoff behandelt.

3. Verfahren nach Anspruch 2, dadurch gekennzeichnet, daß der Rhodium enthaltende Katalysator außer Rhodium wenigstens einen Liganden aus der Gruppe Kohlenmonoxid, der zwei olefinische Doppelbindungen aufweisenden Alkene mit 2 bis 12 C-Atomen, der tertiären Amine, der Disulfide und Sulfoxide, der Acetylacetonate oder der tertiären organischen Phosphine und Phosphite enthält.

4. Verfahren nach Ansprüchen 2 und 3, dadurch gekennzeichnet, daß der Rhodium enthaltende Katalysator dimeres Rhodiumcarbonylchlorid $Rh_2(C_8H_{12})_2Cl_2$, $Rh(CO)_2acac$, $Rh[(C_6H_5CH_2)_2S]_3Cl_3$, $Rh(PPh_3)_3Cl$, $[Rh[C_5H_5)_2Fe]PPh_2]_3Cl_3$, $Rh(PPh_3)_3Br$, $Rh(PPh_3)_3Cl_3$, Rhodium-2-ethylhexanoat, $Rh[(4\text{-}CH_3O\text{-}C_6H_4CH_2)_2S]_3Cl_3$, $Rh[(4\text{-}Cl\text{-}C_6H_4CH_2)_2S]_3Cl_3$, $Rh[(C_6H_5CH_2)_2SO]_3Cl_3$, und $Rh[(CH_3)_2SO]_3Cl_3$ ist. acac steht hierbei für einen Acetylacetonatrest und Ph für eine Phenylgruppe.

5. Verfahren nach Ansprüchen 2 bis 4, dadurch gekennzeichnet, daß der Rhodium enthaltende Katalysator in einer Menge eingesetzt wird, die 10 bis 60 mg Rhodium pro kg Ausgangsprodukt entspricht.

6. Verfahren nach Ansprüchen 2 bis 5, dadurch gekennzeichnet, daß man die Umsetzung mit Kohlenmonoxid und Wasserstoff und die Behandlung mit Wasserstoff in Gegenwart von Ammoniak und einem Hydrierkatalysator in Gegenwart eines Lösungsmittels durchführt.

7. Verfahren nach Ansprüchen 2 bis 6, dadurch gekennzeichnet, daß man die Umsetzung mit Kohlenmonoxid und Wasserstoff bei Temperaturen von 105 bis 180 °C und Drucken von 100 bis 400 bar durchführt.

8. Verfahren nach Ansprüchen 2 bis 7, dadurch gekennzeichnet, daß man die Behandlung des Dihydroformylierungsproduktes des Limonens mit Wasserstoff in Gegenwart von Ammoniak in Gegenwart eines Katalysators durchführt, der als aktive Komponente eines oder mehrere der Elemente mit den Ordnungszahlen 23 bis 29 in metallischer und/oder oxidischer Form enthält.

9. Verfahren nach Ansprüchen 2 bis 8, dadurch gekennzeichnet, daß man die Behandlung des Dihydroformylierungsproduktes des Limonens in Gegenwart von Ammoniak und einem Hydrierkatalysator mit Wasserstoff bei 90 bis 135 °C und einem Wasserstoffdruck von 50 bis 200 bar durchführt.

10. Verwendung von 3-Aminomethyl-1-(3-aminopropyl-1-methyl)-4-methyl-cyclohexan als Korrosionsschutzmittel, Vernetzer für Epoxidharze und Kettenverlängerungsmittel für NCO-Prepolymere.

## Claims

1. 3-Aminomethyl-1-(3-aminopropyl-1-methyl)-4-methyl-cyclohexane.

2. Process for the preparation of 3-aminomethyl-1-(3-aminopropyl-1-methyl)-4-methyl-cyclohexane, characterised in that limonene and/or monohydroformylation products of limonene are reacted with carbon monoxide and hydrogen at temperatures from 80 to 180 °C under pressures from 20 to 1 000 bar in the presence of a rhodium-containing catalyst which, in addition to rhodium, contains at least one ligand from the group comprising carbon monoxide, alkenes with 2 to 12 C atoms and organic compounds containing nitrogen, sulphur, oxygen or phosphorus, in an amount which corresponds to less than 1 g of rhodium per kg of starting material, and the resulting dihydroformylation product of limonene is then treated with hydrogen at 50 to 150 °C in the presence of ammonia and a hydrogenation catalyst.

3. Process according to Claim 2, characterised in that, in addition to rhodium, the rhodium-containing catalyst contains at least one ligand from the group comprising carbon monoxide, alkenes with 2 to 12 C atoms and two olefinic double bonds, tertiary amines, disulphides, sulphoxides, acetylacetonates and tertiary organic phosphines and phosphites.

4. Process according to Claims 2 and 3, characterised in that the rhodium-containing catalyst is dimeric rhodium-carbonyl-chloride, $Rh_2(C_8H_{12})_2Cl_2$, $Rh(CO)_2acac$, $Rh[(C_6H_5CH_2)_2S]_3Cl_3$, $Rh(Pph_3)_3Cl$, $[Rh[C_5H_5)_2Fe]Pph_2]_3Cl_3$, $Rh(Pph_3)_3Br$, $Rh(Pph_3)_3Cl_3$, rhodium 2-ethylhexanoate, $Rh[(4\text{-}CH_3O\text{-}C_6H_4CH_2)_2S]_3Cl_3$, $Rh[(4\text{-}Cl\text{-}C_6H_4CH_2)_2S]_3Cl_3$, $Rh[(C_6H_5CH_2)_2SO]_3Cl_3$ and $Rh[CH_3)_2SO]_3Cl_3$ in

which acac represents an acetylacetonate radical and Ph represents a phenyl group.

5. Process according to Claims 2 to 4, characterised in that the rhodium-containing catalyst is used in an amount which corresponds to 10 to 60 mg of rhodium per kg of starting material.

6. Process according to Claims 2 to 5, characterised in that the reaction with carbon monoxide and hydrogen and the treatment with hydrogen are carried out in the presence of ammonia and a hydrogenation catalyst in the presence of a solvent.

7. Process according to Claims 2 to 6, characterised in that the reaction with carbon monoxide and hydrogen is carried out at temperatures from 105 to 180 °C under pressures from 100 to 400 bar.

8. Process according to Claims 2 to 7, characterised in that the treatment of the dihydroformylation product of limonene with hydrogen is carried out in the presence of ammonia in the presence of a catalyst which contains, as the active component, one or more elements of atomic number 23 to 29 in metallic and/or oxidic form.

9. Process according to Claims 2 to 8, characterised in that the treatment of the dihydroformylation product of limonene with hydrogen in the presence of ammonia and a hydrogenation catalyst is carried out at 90 to 135 °C under a hydrogen pressure of 50 to 200 bar.

10. Use of 3-aminomethyl-1-(3-aminopropyl-1-methyl)-4-methyl-cyclohexane as a corrosion inhibitor, a crosslinking agent for epoxide resins and a chain lengthening agent for NCO prepolymers.

## Revendications

1. Le 3-aminométhyl-1-(3-aminopropyl-1-méthyl)-4-méthyl-cyclohexane.

2. Procédé de préparation du 3-aminométhyl-1-(3-aminopropyl-1-méthyl)-4-méthyl-cyclohexane, caractérisé en ce que l'on fait réagir le limonène et/ou des produits de monohydroformylation du limonène en présence d'un catalyseur contenant du rhodium et qui, en plus du rhodium, contient au moins un ligand du groupe de l'oxyde de carbone, des alcènes en C 2-C 12 et des composés organiques contenant de l'azote, du soufre, de l'oxygène ou du phosphore, en quantité correspondant à moins de 1 g de rhodium par kg du produit de départ, avec de l'oxyde de carbone et de l'hydrogène à des températures de 80 à 180 °C et des pressions de 20 à 1 000 bars puis on traite le produit de dihydroformylation du limonène ainsi obtenu, en présence d'ammoniac et d'un catalyseur d'hydrogénation, par l'hydrogène à une température de 50 à 150 °C.

3. Procédé selon la revendication 2, caractérisé en ce que le catalyseur contenant du rhodium contient, en plus du rhodium, au moins un ligand du groupe de l'oxyde de carbone, des alcènes en C 2-C 12 contenant deux doubles liaisons oléfiniques, des amines tertiaires, des disulfures et des sulfoxydes, des acétylacétonates ou des phosphines et phosphites organiques tertiaires.

4. Procédé selon les revendications 2 et 3, caractérisé en ce que le catalyseur contenant du rhodium est le chlorure de rhodium-carbonyle dimère $Rh_2(C_8H_{12})_2Cl_2$, $Rh(CO)_2acac$, $Rh[(C_6H_5CH_2)_2S]_3Cl_3$, $Rh(PPh_3)_3Cl$, $[Rh[(C_5H_5)_2Fe]PPh_2]_3Cl_3$, $Rh(PPh_3)_3Br$, $Rh(PPh_3)_3Cl_3$, le 2-éthylhexanoate de rhodium, $Rh[(4\text{-}CH_3O\text{-}C_6H_4CH_2)_2S]_3Cl_3$, $Rh[(4\text{-}Cl\text{-}C_6H_4CH_2)_2S]_3Cl_3$, $Rh[C_6H_5CH_2)_2SO]_3Cl_3$ et $Rh[(CH_3)_2SO]_3Cl_3$, acac représentant dans ces formules un reste acétylacétonate et Ph un groupe phényle.

5. Procédé selon les revendications 2 à 4, caractérisé en ce que le catalyseur contenant du rhodium est utilisé en quantité correspondant à 10 à 60 mg de rhodium par kg du produit de départ.

6. Procédé selon les revendications 2 à 5, caractérisé en ce que la réaction avec l'oxyde de carbone et l'hydrogène et le traitement par l'hydrogène en présence d'ammoniac et d'un catalyseur d'hydrogénation sont effectués en présence d'un solvant.

7. Procédé selon les revendications 2 à 6, caractérisé en ce que la réaction avec l'oxyde de carbone et l'hydrogène est effectuée à des températures de 105 à 180 °C et des pressions de 100 à 400 bars.

8. Procédé selon les revendications 2 à 7, caractérisé en ce que le traitement du produit de dihydroformylation du limonène par l'hydrogène en présence d'ammoniac est effectué en présence d'un catalyseur contenant en tant que composant actif un ou plusieurs des éléments de numéro atomique 23 à 29 à l'état de métal et/ou d'oxyde.

9. Procédé selon les revendications 2 à 8, caractérisé en ce que le traitement du produit de dihydroformylation du limonène en présence d'ammoniac et d'un catalyseur d'hydrogénation est effectué avec de l'hydrogène à une température de 90 à 135 °C et une pression d'hydrogène de 50 à 200 bars.

10. Utilisation du 3-aminométhyl-1-(3aminopropyl-1-méthyl)-4-méthyl-cyclohexane en tant qu'agent anticorrosion, réticulant pour résines époxydiques et agent d'allongement des chaînes pour prépolymères à groupes NCO.